Europäisches Patentamt

(19) European Patent Office

Office européen des brevets

(11) Veröffentlichungsnummer: **0 074 050**
**B1**

(12) EUROPÄISCHE PATENTSCHRIFT

(45) Veröffentlichungstag der Patentschrift:
04.12.85

(51) Int. Cl.⁴: **A 61 K 9/14, A 23 L 1/30**

(21) Anmeldenummer: **82107951.4**

(22) Anmeldetag: **30.08.82**

(54) Verfahren zur Herstellung von Trockenpulvern oxidationsempfindlicher Substanzen.

(30) Priorität: **05.09.81 DE 3135329**

(43) Veröffentlichungstag der Anmeldung:
**16.03.83 Patentblatt 83/11**

(45) Bekanntmachung des Hinweises auf die Patenterteilung:
**04.12.85 Patentblatt 85/49**

(84) Benannte Vertragsstaaten:
**CH DE FR GB IT LI**

(56) Entgegenhaltungen:
**EP - A - 0 005 529**
**CH - A - 420 817**
**CH - A - 431 252**
**US - A - 3 962 384**

(73) Patentinhaber: **BASF Aktiengesellschaft,**
**Carl-Bosch-Strasse 38, D-6700 Ludwigshafen (DE)**

(72) Erfinder: **Schumacher, Horst, Battenberger Weg 4,**
**D-6719 Bobenheim (DE)**
Erfinder: **Grafen, Paul, Dr., Suedtiroler Ring 18-20,**
**D-6719 Weisenheim (DE)**

## Beschreibung

Die Erfindung betrifft ein verbessertes Verfahren zur Herstellung von Trockenpulvern, insbesondere öliger, oxidationsempfindlicher Substanzen, durch Versprühen einer Kolloiddispersion der öligen Substanzen, unter Zugabe von Sprühhilfsmitteln.

Es gibt verschiedene Sprühverfahren, um oxidationsempfindliche Stoffe wie die Vitamine A, E und D oder Carotinoide in trockene, gut rieselfähige, gegen oxidative Einflüsse geschützte Pulver zu überführen. Das Pulver soll aus Teilchen mit gut ausgebildeter Oberfläche in einer Korngröße von 100 bis 600 µm, mit einem Maximum bei etwa 250 µm, bestehen. Pulver mit diesem Korngrößenbereich der Teilchen gewährleisten einen ausreichenden Schutz des Wirkstoffes und die Anzahl der Partikel je Gewichtseinheit ist noch groß genug, damit in der weiterverarbeitenden pharmazeutischen Industrie, Nahrungs- und Futtermittelindustrie eine homogene Mischung dieser Produkte mit anderen Stoffen oder mit Nahrungs- und Futtermitteln möglich ist.

Bei den bekannten Verfahren, die zu Pulvern mit den gewünschten Eigenschaften führen, wird z. B. eine wäßrige Dispersion des öligen Wirkstoffes mit einem filmbildenden Kolloid aus der Gruppe der Proteine, wie Gelatine und Kasein oder der Polysaccharide, wie Pektine, Gummiarabikum oder Celluloseverbindungen und gegebenenfalls einem Zucker oder Zuckeralkohol wie Glucose, Laktose, Saccharose, Sorbitol, meist unter weiterem Zusatz von Antioxidantien, Emulgatoren und/oder Konservierungsmitteln hergestellt, die Dispersion versprüht und das Sprühgut anschließend getrocknet.

Im einzelnen ist beispielsweise aus der deutschen Patentschrift 1 035 319 bekannt, eine Dispersion eines öligen Vitamins in einen großen Überschuß eines Stärkepulvers mit einem Wassergehalt unter 8% zu versprühen. Das trockene Stärkepulver entzieht den Partikeln soviel Wasser, daß sie erstarren. Ein großer Nachteil dieses Verfahrens besteht darin, daß etwa 15% der Stärkemenge an der Oberfläche der Partikel haften bleiben und der überschüssige Stärkeanteil abgetrennt werden muß, damit er nach dem Trocknen wieder dem Prozeß zugeführt werden kann.

Nach einem anderen Verfahren, das in der schweizerischen Patentschrift 488 455 beschrieben ist, wird die Stärke durch ein Gemisch wasserabsorbierender und wassernichtabsorbierender anorganischer Substanzen ersetzt, um die Explosionsgefahr, die von der feinverteilten Stärke ausgeht, auszuschalten. Für optimale Ergebnisse ist hier sogar ein 20facher Überschuß des Auffangpulvers notwendig.

Gemäß dem Verfahren der schweizerischen Patentschrift 389 505 wird die Wirkstoffdispersion in ein gekühltes, gasförmiges Medium gesprüht, in dem die Partikel bis zur Erstarrung verweilen. Dies erfordert in der Regel Fallhöhen von 12 bis 15 m bei Temperaturen unterhalb der Raumtemperatur und ist aus wirtschaftlichen Gründen oft nur mit hochschmelzenden Kolloiden durchführbar.

Weiterhin ist in der schweizerischen Patentschrift 420 817 ein Verfahren beschrieben, bei dem das Zusammenkleben der in gekühlte Luft gesprühten Teilchen dadurch verhindert wird, daß man auf ein sich bezüglich der Sprühdose schnell bewegendes, speziell beschichtetes Band sprüht, von welchem die erstarrten Teilchen durch ein Schab- oder Abbürstesystem entfernt werden, um anschließend getrocknet zu werden.

Schließlich ist das Verfahren der schweizerischen Patentschrift 431 252 zu erwähnen, gemäß der die Dispersion bei einer Temperatur, bei der die Partikel erstarren, in ein Auffangpulver gesprüht wird, das aus einem Gemisch aus erstarrten und erstarrenden Partikeln und einem Metallsalz einer höheren Fettsäure als Gleitmittel, zusammengesetzt ist, wobei der Boden der Auffangkammer aus einem speziell aufgebauten Siebsystem bestehen muß. Auch hier schließt sich die Trocknung an.

Alle diese Verfahren befriedigen nicht. Es bestand daher die Aufgabe, ein Verfahren vorzuschlagen, das es gestattet, in einfacher Weise oxidationsempfindliche, insbesondere ölige Wirkstoffe in feinteilige Pulver zu überführen.

Diese Aufgabe wurde erfindungsgemäß gelöst durch Dispergieren von öllöslichen Stoffen wie Vitaminen, Carotinoiden, pharmazeutischen Wirkstoffen oder Aromastoffen in einer wäßrigen Lösung eines filmbildenden Kolloids, wobei das Kolloid die homogene Phase der Dispersion darstellt, unter Zusatz eines oder mehrerer Stoffe aus der Gruppe der Mono-, Di- oder Polysaccharide, Versprühen der Dispersion in einem Sprühturm unter Mitverwendung eines Sprühhilfsmittels und Auffangen der versprühten Teilchen in einem Fließbett, wobei man als Sprühhilfsmittel eine hydrophobe Kieselsäure oder das Metallsalz einer höheren Fettsäure, z. B. mit 16 bis 18 C-Atomen oder Gemische mit hydrophober Kieselsäure, in der 0,02 bis 0,15fachen Gewichtsmenge, bezogen auf die Dispersion (und in Abwesenheit wesentlicher Mengen anderer üblicher Sprühhilfsmittel wie Stärkepulver) oberhalb des Fließbetts unter gleichmäßiger Verteilung in den Sprühraum einführt, bei Temperaturen, bei denen eine Erstarrung des gegebenenfalls gelierenden Kolloids der versprühten Teilchen noch nicht eintritt, die mit dem Sprühhilfsmittel beladenen Teilchen, deren Kolloidmasse im wesentlichen nicht geliert ist, in einem Fließbett auffängt und die Teilchen in an sich bekannter Weise im Fließbett trocknet.

Als Kolloide kommen bevorzugt Gelatine, beispielsweise 70 bis 200 Bloom oder Casein in Betracht. Die Menge des angewandten Kolloids beträgt in der Regel 5 bis 50 Gew.-%, bezogen auf das Endprodukt bei Wassergehalten der Dispersion von 30 bis 70 Gew.-%. Zur Herstellung der Dispersion werden die Filmbildner und anschließend die Wirkstoffe in der 50 bis 70°C warmen Zuckerlösung dispergiert. Die Dispersion wird dann zerstäubt.

Die Ausbildung des Zerstäubungsaggregats hat keinen entscheidenden Einfluß auf das Produkt. So können beispielsweise Düsen oder schnell rotierende Zerstäuberscheiben benutzt werden. Die Temperatur der zu zerstäubenden Dispersion ist ebenfalls keine kritische Größe. Sie liegt üblicherweise bei 60 bis 90°C, das ergibt bei den genannten Kolloiden Viskositäten von 50 bis 1200 mPas (60°C). Entscheidend ist, daß zum Zeitpunkt des Versprühens die Partikel mit dem hydrophoben Sprühhilfsmittel in Kontakt kommen, das in feinverteilter Form direkt in die Sprühzone eingeführt wird.

Der große Vorteil des neuen Verfahrens besteht darin, daß die Temperatur im Sprühraum nicht mehr so tief liegen muß, daß eine Gelbildung der Wirkstoffdispersion eintritt, oder daß nicht mehr durch große Mengen an Hilfspulver soviel Wasser entzogen werden muß, daß eine Erstarrung der Tröpfchen erfolgt. Das neue Verfahren ermöglicht beispielsweise das Versprühen bei Temperaturen von 25 bis 30°C von Wirkstoffdispersionen, die selbst bei Kühlschranktemperaturen (+4°C) nicht mehr erstarren. Die dazu notwendigen Mengen des Sprühhilfsmittels betragen hierbei nur das 0,02 bis 0,15fache der Dispersion.

Als hydrophobe Sprühhilfsmittel kommen silanisierte Kieselsäuren, wie sie in »Die Mühle und Mischfuttertechnik« 114, 3 (1977) beschrieben sind oder Metallsalze der höheren Fettsäuren mit 16 bis 18 C-Atomen wie Calcium- oder Magnesiumstearat oder deren Mischung mit hydrophober Kieselsäure in Betracht. Durch das direkte Einbringen in die Sprühzone wird die mechanische Beanspruchung der Partikel weitgehend vermieden, die beispielsweise von einem Träger-gefüllten Fluidbett ausgeht. Der während des Sprühens erzeugte dünne hydrophobe Film des Sprühhilfsmittels stabilisiert die Partikel soweit, daß ein Zusammenlaufen der Partikel bei Berührung im nicht erstarrten Zustand verhindert wird, so daß die direkte Trocknung auf einem sich anschließenden Wirbelbett-Trockner möglich ist.

Die Zufuhr des Sprühhilfsmittels und dessen Zerstäubung erfolgt zusammen mit Luft bei Raumtemperatur mit etwa 5 m³/kg Sprühhilfsmittel, zweckmäßig oberhalb des Zerstäubungsaggregats.

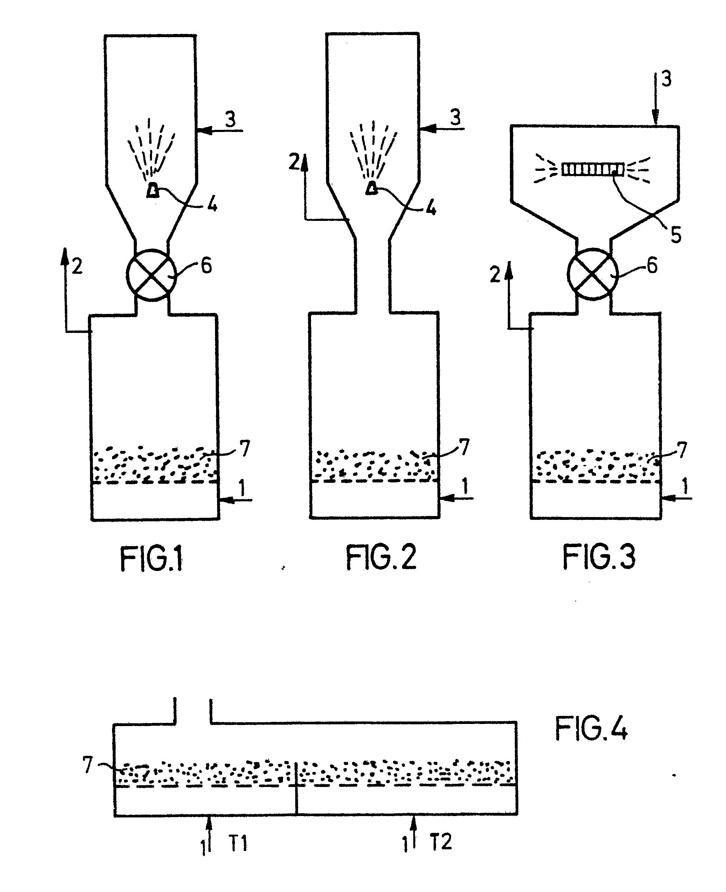

Das erfindungsgemäße Verfahren kann wie in den Fig. 1 bis 4 beschrieben, wie folgt ausgeführt werden.

Die Fig. 1 bis 3 stellen Vorrichtungen mit diskontinuierlicher Trocknung der Pulver dar. Fig. 4 zeigt eine Vorrichtung mit kontinuierlicher Trocknung. Darin bedeuten (1) Trockenluftzufuhr, (2) Trockenluftabfuhr, (3) Sprühhilfsmittelzugabe (Injektor), (4) eine Zerstäubungsdüse, (5) einen Sprühteller, (6) ein Zellenrad, (7) das Wirbelbett,

$T_1$ eine Temperatur von 20 bis 40°C und $T_2$ eine Temperatur von 40 bis 90°C.

Im einzelnen ist das neue Verfahren in den folgenden Beispielen beschrieben:

### Beispiel 1
### (Anordnung gemäß Fig. 1)

In eine Lösung von 56,4 Teilen Glucosesirup (80% Feststoffgehalt) in 49 Teilen Wasser werden 14 Teile Gelatine (100 Bloom) eingerührt und 1 Stunde quellen gelassen. Man gibt darauf 13,6 Teile Maisstärke zu und emulgiert bei 61 bis 63°C 24,9 Teile Vitamin A-Acetat (2,21 Mio I.E./g), stabilisiert mit Ethoxyquin, ein. Die erhaltene Dispersion mit einer Viskosität von 104 mPas (60°C) wird bei einer Temperatur von 80 bis 90°C und 60 bar Sprühdruck versprüht. Gleichzeitig gibt man während des Sprühens in die Sprühzone hydrophobe Kieselsäure (Sipernat D17) in Mengen von 10,5 kg/h. Die Sprühleistung beträgt 162 kg/h Dispersion. Nach der Trocknung in einem Wirbelbett bei 28 bis 38°C erhält man ein Pulver der folgenden Zusammensetzung:

Gehalt 572 000 I.E./g

| Siebanalyse nach DIN | | |
|---|---|---|
| | 500 µm | 3,5% |
| | 400 µm | 9,8% |
| | 250 µm | 38,9% |
| | 160 µm | 35,0% |
| | 125 µm | 8,5% |
| | 100 µm | 2,7% |

### Beispiel 2
### (Anordnung gemäß Fig. 3)

Man verfährt wie in Beispiel 1 beschrieben, zerstäubt die Dispersion jedoch bei 62°C mit einer Viskosität von 1196 mPas (60°C) drucklos mit Hilfe einer Zerstäuberscheibe (5) bei 24 300 U/Min. Die Sprühleistung beträgt 77 kg/h Dispersion bei einem Einsatz von 4,5 kg/h hydrophober Kieselsäure (Sipernat D 17). Der Vitamingehalt des Pulvers beträgt 591 000 I.E./g, die Siebanalyse nach ASTM

| Nr. | | |
|---|---|---|
| | 35 | 0,2% |
| | 40 | 1,4% |
| | 45 | 12,3% |
| | 60 | 46,0% |
| | 80 | 30,2% |
| | 120 | 7,5% |
| | 140 | 1,4% |

### Beispiel 3
### (Anordnung gemäß Fig. 1)

In eine Lösung aus 53 Teilen Wasser und 7,9 Teilen Saccharose werden 19,4 Teile Gelatine (100 Bloom) eingerührt und 1 Stunde quellen gelassen. Man erwärmt danach auf 62°C, emulgiert 6,8 Teile Vitamin A-Acetat (2,71 Mio. I.E./g) ein und stabilisiert mit Butylhydroxytoluol. Die Dispersion hat eine Viskosität von 127 mPas (60°C) und wird bei 80 bis 90°C mit einer Einstoffdüse (4) bei einem Druck von 50 bar versprüht. Während des Sprühens werden 22 kg/h Calciumstearat (Ceasit levissimum) in die Sprühkammer mit einem Luftstrom zugeführt. Die Sprühleistung an Dispersion beträgt 260 kg/h. Die Trocknung des Produktes erfolgt im Wirbelbett bei 28°C. Der Vitamingehalt des Pulvers beträgt 514 000 I.E./g. Die Siebanalyse nach ASTM ergibt:

| Nr. | 30 | < 1% |
|---|---|---|
| | 35 | 5,7% |
| | 40 | 15,7% |
| | 45 | 21,2% |
| | 60 | 28,7% |
| | 80 | 17,8% |
| | 120 | 7,9% |
| | 140 | 2,0% |

### Beispiel 4
### (Anordnung gemäß Fig. 2)

Man rührt in eine 63°C warme Lösung aus 15 Teilen Wasser und 39,5 Teilen Glucosesirup (80% Feststoffgehalt) 2,8 Teile Säurecasein (110 mesh) ein. Durch Zugabe von 10%iger Natronlauge wird der pH-Wert auf 7 eingestellt. Anschließend emulgiert man 0,25 Teile Fettsäuremonoglycerid und 12,8 Teile Vitamin A-Acetat (2,18 Mio I.E./g), stabilisiert mit Ethoxyquin, ein. Die Dispersion wird mit einer Viskosität von 202 mPas (bei 60°C) bei 80 bis 90°C mit einer Einstoffdüse und bei einem Druck von 50 bar versprüht. Man gibt während des Sprühens 18,5 kg/h hydrophobe Kieselsäure (Sipernat D 17) in die Sprühzone. Die Sprühleistung an Dispersion beträgt 150 kg/h. Man trocknet das Produkt mit einem Wirbeltrockner bei 25 bis 33°C innerhalb 7 Stunden. Vitamingehalt des Pulvers 542 000 I.E./g.; Siebanalyse nach ASTM:

| Nr. | 35 | 0,2% |
|---|---|---|
| | 40 | 0,2% |
| | 45 | 1,1% |
| | 60 | 24,1% |
| | 80 | 49,3% |
| | 120 | 17,8% |
| | 140 | 4,2% |

### Beispiel 5
### (Anordnung gemäß Fig. 2)

Man rührt in eine Lösung aus 50 Teilen Wasser und 25 Teilen Dextrose 9 Teile Gelatine ein und erwärmt nach Zugabe von 9 Teilen Stärke auf 62°C. In dieser Mischung dispergiert man 7,5 Teile einer Cantaxanthinmischung mit Pflanzenfett und Ethoxyquin (Wirkstoffgehalt 70%). Die Viskosität der Dispersion beträgt 163 mPas (60°C). Die Dispersion wird bei 62°C unter Zudosierung von 11 kg/h hydrophober Kieselsäure (Sipernat D 17) in den Sprühraum gegeben bei einer Sprühleistung an Dispersion von 295 kg/h. Die Trocknung erfolgt im Wirbelbett bei 28 bis 30°C. Man erhält ein Trockenpulver mit einem Wirkstoffgehalt von 9,8% und einer Siebanalyse nach ASTM von

| Nr. | 35 | 0,2% |
|---|---|---|
| | 40 | 0,8% |
| | 45 | 5,9% |
| | 60 | 43,7% |
| | 80 | 31,2% |
| | 120 | 11,5% |
| | 140 | 3,5% |

### Patentansprüche

1. Verfahren zur Herstellung von trockenen, freifließenden Pulvern leicht oxidierbarer Stoffe, wie der Vitamine, Carotinoide oder Aromastoffe, die von einem Kolloid eingehüllt sind, durch Dispergieren dieser Stoffe in einer wäßrigen Lösung eines filmbildenden Kolloids, wobei das Kolloid die homogene Phase darstellt, unter Zusatz eines oder mehrerer Stoffe aus der Gruppe der Mono-, Di- oder Polysaccharide, Versprühen der Dispersion in einem Sprühturm unter Mitverwendung eines Sprühhilfsmittels und Auffangen der versprühten Teilchen in einem Fließbett, dadurch gekennzeichnet, daß man als Sprühhilfsmittel eine hydrophobe Kieselsäure oder ein Metallsalz einer höheren Fettsäure oder Gemische mit hydrophober Kieselsäure, in der 0,02- bis 0,15fachen Gewichtsmenge, bezogen auf die Dispersion und in Abwesenheit wesentlicher Mengen anderer üblicher Sprühhilfsmittel wie Stärkepulver, oberhalb des Fließbetts unter gleichmäßiger Verteilung in den Sprühraum bei Temperaturen einführt, bei denen eine Erstarrung des Kolloids der versprühten Teilchen noch nicht eintritt, die mit Hilfsmittel beladenen Teilchen, deren Kolloidmasse im wesentlichen nicht geliert ist, in einem Fließbett auffängt und die Teilchen in an sich bekannter Weise im Fließbett trocknet.

2. Verfahren gemäß Anspruch 1, dadurch gekennzeichnet, daß man als Kolloid Gelatine oder Casein verwendet.

3. Verfahren gemäß Anspruch 1, dadurch gekennzeichnet, daß man die zur Trocknung dem Fließbett zugeführte Luft unterhalb der Sprühzone aus dem Sprühturm abzieht.

### Claims

1. A process for the production of a dry, free-flowing powder of a readily oxidizable substance, eg. a vitamin, a carotinoid or an aroma, which is coated with a colloid, by dispersing this substance in an aqueous solution of a film-forming colloid, which constitutes the homogenous phase, with the addition of one or more substances selected from the group consisting of the mono-, di- and polysaccharides, atomizing the dispersion in a spray tower in the presence of a spraying assistant, and collecting the resulting particles in a fluidized bed, wherein, as the spraying assistant, from 0.02 to 0.15 times the amount by weight, based on the dispersion, of a hydrophobic silica or a metal salt of a higher fatty acid, or a mixture of such a salt with silica, is introduced — in the absence of significant amounts of other spraying assistants, such as starch powder — above the fluidized bed and distributed uniformly in the spraying space, at a temperature at which the colloid of the atomized particles has not yet solidified, and the assistant-laden particles, the colloid material of which has essentially not gelatinized, is collected in a fluidized bed, and dried therein in a conventional manner.

2. A process as claimed in claim 1, wherein gelatin or casein is used as the colloid.

3. A process as claimed in claim 1, wherein the air fed to the fluidized bed for drying purposes is removed below the spraying zone of the spray tower.

## Revendications

1. Procédé pour la préparation de poudres sèches, s'écoulant librement, de substances facilement oxydables, telles que vitamines, caroté-noïdes ou substances aromatiques, qui sont enrobées d'un colloïde, par dispersion de ces substances dans une solution aqueuse d'un colloïde filmogène, le colloïde représentant la phase homogène, avec addition d'une ou de plusieurs substances du groupe des mono-, di- ou polysaccharides, pulvérisation de la dispersion dans une tour de pulvérisation avec utilisation simultanée d'un adjuvant de pulvérisation et captage des particules pulvérisées dans un lit fluidisé, caractérisé en ce qu'on introduit dans la zone de pulvérisation, au-dessus du lit fluidisé et avec distribution simultanée, en tant qu'adjuvant de pulvérisation, un acide silicique hydrophobe, un sel métallique d'un acide gras supérieur ou des mélanges de ce sel avec un acide silicique hydrophobe, dans une proportion en poids de 0,02 à 0,15 fois par rapport à la dispersion et en l'absence de quantités importantes d'autres adjuvants de pulvérisation usuels tels que la poudre d'amidon, à des températures auxquelles une solidification du colloïde des particules pulvérisées ne se produit pas encore, on capte dans un lit fluidisé les particules chargées d'adjuvant, dont la masse colloïdale n'est pratiquement pas gélifiée et on sèche les particules de façon connue en soi dans le lit fluidisé.

2. Procédé selon la revendication 1, caractérisé en ce qu'on utilise des gélatines ou une caséine en tant que colloïde.

3. Procédé selon la revendication 1, caractérisé en ce qu'on extrait de la tour de pulvérisation, au-dessous de la zone de pulvérisation, l'air introduit dans le lit fluidisé pour le séchage.

FIG.1      FIG.2      FIG.3

FIG.4